# EUROPEAN PATENT APPLICATION

(11) **EP 4 632 169 A1**
(43) Date of publication of application: **15.10.2025**
(21) Application number: 24169387.8
(22) Date of filing: 10.04.2024
(51) Int. Cl.: E04D 13/00, G01N 22/04, G01N 33/00, G01K 11/00, G01N 33/44, G08B 21/20, H01Q 21/06

(54) **METHOD FOR DETECTING MOISTURE IN A ROOF STRUCTURE**

(71) Applicant: Sika Technology AG, 6340 Baar (CH)
(72) Inventor: HOLDENER, Sabina, 6105 Schachen (CH); VON BERGEN, Eduard, 6060 Sarnen (CH); CARL, Wilfried, 8280 Kreuzlingen (CH); DARSCH, Michael, Lakeville, MA 02347 (US); LEEBER, Blaise W., Burlington, MA 01803 (US); PADWAL, Ajay, Sharon, MA 02067 (US); ROHRER, Martin, 6072 Sachseln (CH)
(74) Representative: Sika Patent Attorneys

(57) **Abstract**

The invention is directed to a method for detecting moisture in a roof structure comprising measuring microwave emission of the roof structure, particularly by using a passive microwave radiometer. The invention is also directed to use of a passive microwave radiometer for detecting leakages in a roof structure.

## Description

### Technical field

The invention relates to detecting moisture in roof structures using a non-invasive method based on measuring microwave emission of the roof structure. The invention also relates to use of a passive microwave radiometer for detecting of leakages in a roof structure.

### Background Art

Flat roofs are commonly waterproofed against the penetration of water by using prefabricated polymeric single- and multi-ply roofing membranes, which are often based on thermoplastic materials such as TPOs (thermoplastic olefins) and plasticized polyvinylchloride (p-PVC) or crosslinked elastomers, particularly ethylene propylene diene monomer (EPDM). An alternative to such prefabricated membranes are liquid applied membranes (LAMs) which are based on 1-component or 2-component reactive compositions, for example, on the basis of polyurethanes (PUs) or silicones, which cure after their application in order to gain their final physical properties.

Polymeric membranes have a general disadvantage of having relatively low resistance to mechanical impacts caused by objects falling on the roof. Furthermore, roofing membranes form the outermost protection layer of a roof system and, therefore, the material of the membrane is constantly exposed to varying environmental conditions including rapid temperature changes, excessive amount of moisture/water, and UV radiation resulting in physical and chemical changes in the material of the membrane, also known as "aging".

Once a roofing membrane has been damaged, it cannot anymore perform its waterproofing function, which results in the ingress of water into the building with subsequent damage to the building structure and goods inside the building. Ideally all leakages should be detected as soon as possible after the occurrence of the damage so that the amount of water infiltrated into the roof structure can be minimized.

Various methods are available on the market for detecting leakages in roof structures. Most leak detection methods are based on measuring a change in one or more physical properties, such as electrical resistance/conductance, capacitance, optical properties, or electromagnetic properties. The methods currently available on the market are either based on one-time measurements conducted on the site or on the use of stand-alone measurement equipment, which is permanently integrated into the roof structure, such as humidity sensors.

Each of the commonly used method has some limitations, or sometimes such a high degree of sophistication that use on ,every-day-roofs' is simply not justified. Particularly, installation of embedded moisture sensors requires a power supply, either by a permanent supply or by a battery. While permanent power supply is rarely available on typical commercial flat roofs, use of batteries requires regular change or re-charging, which can be very difficult for embedded systems. Another method for detecting water infiltration/moisture in roof structures is based on the use of thermal imaging cameras, also known as thermographic infrared measurements. Thermal imaging detects the change of heat capacity caused by a wet insulation layer, which leads to a delayed heating up or cooling down, compared to a dry roof. This method has the drawback that it requires heating and/or cooling phases, which means that the measurement must typically to be carried out after sunrise or sunset.

The methods based on use humidity sensors and thermographic infrared measurements are also prone false alarms since the presence of water in the roof structure does not necessary indicate that there is a leakage in the roofing membrane. During the cold season, some moisture might condense and accumulate in the roof structure without the presence of a leakage. Both measurement techniques also suffer from the common disadvantage that even though the presence of moisture can be detected, the exact location of the leakage must be conducted by a trained person. Consequently, the leakages are typically discovered only after the water has already caused significant damage to the building structures.

Typically, the costs of leakage detection are so high that their use is economically justified only after the leakage has already been detected by other means. For example, in the high and low voltage electric conductance methods as disclosed in ASTM D7877-14 standard, a conductive substrate (conductor) has to be installed under the roofing membrane to serve as a ground return path for the test currents. In a low voltage method, the top surface of the membrane is covered by a water film, which forms the other conductor whereas in a high voltage method, another conductor is installed above the membrane. In both cases the method can only be conducted by highly trained personnel. Furthermore, in many cases identifying the exact location of the leakage is not possible even with these high cost methods.

Still another method for determining moisture in a roof structure is based on the measurement of the dielectric constant. This method requires close contact between the roof surface and the measuring equipment and it is also sensitive towards several disturbing factors. Moreover, the signal is weak and depends strongly on the dielectric properties of the membrane, for example, PVC vs. TPO.

There thus remains a need for a reliable, robust, and cost efficient method for detecting moisture in roof structures, which method can be carried out by non-destructive means using an external "scanning" method that does not require the use of any pre-installed electric devices or highly trained personnel.

### Summary of the invention

The object of the present invention is to provide a non-destructive, reliable, robust, and cost efficient method for detecting moisture in roof structures.

Surprisingly, it was discovered that the object can be achieved with the features of claim 1.

Specifically, according to the invention, a method for detection of moisture in a roof structure is provided, the method comprising measuring microwave emission of the roof structure.

As it turned out, the proposed method provides a non-destructive, reliable, robust, and cost efficient technique for detecting moisture in roof structures, which method can be used for identifying and locating leakages. The microwave emission of the roof structure is preferably measured using a passive microwave radiometer, which can be installed, for example, on an unmanned aerial vehicle (UAV), a movable cart, or a hand held device to provide a tool, which can be used for manual, semi-automated of fully automated measurement of moisture content and detection of leakages in the roof structure.

One of the advantages of the suggested method is that it does not require a close contact with the surface of the roof, such as the dielectric methods, or specific conditions (sunset or sunrise) like the thermographic infrared methods discussed above.

Additional aspects of the present invention are defined in further independent claims. Particularly preferred embodiments are outlined throughout the description and the dependent claims.

### Brief description of the Drawings

Fig. 1 shows brightness temperatures (TBH, TBV) measured using a portable L-band microwave radiometer in the 1^{st} experimental set-up.
Fig. 2 shows brightness temperatures (TBH, TBV) measured using a portable L-band microwave radiometer in the 2^{nd} experimental set-up.
Fig. 3 shows brightness temperatures (TBH, TBV) measured using a portable L-band microwave radiometer in the 3^{rd} experimental set-up.
Fig. 4 shows brightness temperatures (TBH, TBV) measured using a portable L-band microwave radiometer in the 4^{th} experimental set-up.

### Detailed description of the invention

A first aspect of the present invention is directed to a method for detection of moisture in a roof structure, the method comprising measuring microwave emission of the roof structure.

The term "microwave" refers to a form of electromagnetic radiation having wavelengths shorter than other radio waves but longer than infrared waves, ranging from about one meter to one millimeter, corresponding to frequencies between 300 MHz and 300 GHz, respectively.

Since all objects having a temperature other than absolute zero emit low level microwave black-body radiation with the spectrum and intensity of the emission determined by their physical temperature and structure, a temperature of an object can be determined by measuring its microwave radiant flux, i.e., microwave emission. The devices used for measuring microwave emission are known as microwave radiometers.

According to one exemplary embodiment, the measurement of microwave emission of the roof structure is conducted using a passive microwave radiometer. The use of "a passive radiometer" refers in the present disclosure to a technique whereby the measurements of the temperature profile of an object are achieved without the use of an active transmitter.

Particularly, the passive microwave radiometer may be a portable passive radiometer. The term portable is understood to mean that the radiometer is not fixed to a non-movable structure, such as on a tower, poles, or buildings.

Furthermore, the portable passive radiometer may be integrated into a movable device, such as an unmanned aerial vehicle (UAV), movable cart, or a hand-held device. Suitable portable passive radiometers for these embodiments may have a weight of not more than 20 kg, such as not more than 15 kg, especially not more than 10 kg.

The movable device may further include a navigation module configured to determine the location of the movable device and to navigate it to designated locations. The navigation module may include a global positioning system (GPS) receiver for receiving GPS location signals and determining a location.

Especially, the passive microwave radiometer may be a L-band radiometer, preferably a portable L-band radiometer. Generally the L-band radiometers detect microwaves in a frequency range from 1 to 2 GHz and a wavelength range from 15 to 30 cm. They are typically used for remote sensing of environmental parameters, such as soil moisture, sea surface salinity, snow liquid water content, snow density, vegetation optical depth and others.

In one exemplary embodiment, the measurement of microwave emission of the roof structure is conducted using a passive L-band microwave radiometer, preferably a portable passive L-band microwave radiometer, sensing natural emission in the passive-protected microwave spectrum of 1400 - 1427 MHz.

In one preferred embodiment, the passive microwave radiometer is a dual-polarization L-band radiometer, preferably a portable dual-polarization L-band radiometer.

The term "dual polarization radiometer" refers in the present disclosure to a radiometer having two (vertical and horizontal) polarization antennas for providing two simultaneous and independent measurements.

In one exemplary embodiment, the method comprises measuring dual polarization microwave brightness temperatures and using retrieval algorithms based on microwave emission models to determine the moisture content in the roof structure. The term "brightness temperature" refers here to the measured radiation at the specific frequency (channel).

In one exemplary embodiment, the brightness temperatures are measured at nadir angles at horizontal and vertical polarizations.

Established retrieval algorithms are commonly used for estimating soil water content and other environmental parameters from passive microwave data (brightness temperatures). The retrieval algorithms are based the use of emission models, such as tau-omega (TO) and multiple-scattering two-stream (2S) models. The emission models typically differ from each other on how the radiative transfer mechanisms are considered within the model, particularly, how the radiation emitted by a volume within the vegetation towards the soil contributes to the measured brightness temperatures at the nadir angle and at horizontal and vertical polarizations. For example, in tau-omega emission model, the contribution of the radiation emitted by a volume within vegetation is represented as the radiation reflected at the soil surface and attenuated by the vegetation via absorption and scattering out of the propagating stream, whereas the 0^{th} order one stream model represents vegetation volume scattering as a loss mechanism only, but in addition, considers multiple reflections between the vegetation and the soil surface.

Particularly, the passive radiometer comprises at least one antenna, for example, at least one standard patch antenna, horn antenna, colinear dipole array antenna, or circular polarized or mesh backfire antenna, and a radiometer electronics with radio-frequency components and signal processing components.

In one preferred embodiment, the passive microwave radiometer comprises at least one patch array antenna, preferably comprising at least one patch array, especially two patch arrays, preferably rotated 90 ° relative to each other, wherein one patch array receives horizontal polarized microwaves (H) and the other receives vertical polarized microwaves (V).

In one exemplary embodiment, each patch array of the antenna includes a patch substrate layer as printed circuit board of a dielectric material and a pattern of printed patches on a front side of the patch substrate layer. It may furthermore be preferred that each patch array comprises a sandwich structure of the patch substrate layer, an air gap, and a ground conductor layer.

In a further exemplary embodiment, the passive microwave radiometer comprises a patch array antenna including two patch arrays that are sharing one patch substrate layer and one ground conductor layer, wherein the patterns are printed on the front side of the same patch substrate layer but rotated by °90 to each other. It may further be preferred that the patch array antenna has a dimension of 25 - 100 cm, particularly 35 - 85 cm (length) and 15 - 85 cm, particularly 25 - 75 cm (width).

Preferably, the passive microwave radiometer further comprises a radiometer electronics with radio-frequency components, signal processing components, and a computer unit for collecting and saving the detector data.

Suitable portable passive radiometers for use in the method comprising at least one patch array antenna are commercially available, for example, from Terrarad Tech AG.

In one exemplary embodiment, the passive microwave radiometer is integrated into as an unmanned aerial vehicle (UAV), preferably an aerial drone.

The unmanned aerial vehicle, preferably an aerial drone, may further include at least one camera, at least one on-board communication unit, and optionally a navigation module configured to determine the location of the unmanned aerial vehicle and to navigate it to designated locations. The navigation module may include a global positioning system (GPS) receiver for receiving GPS location signals and determining a location.

The at least one camera is preferable adapted to be part of the aerial drone's flight system. This means the recordings of the camera are used for flying of the aerial drone and/or directing the aerial drone's movement.

In one embodiment, the aerial drone comprises at least one on-board communication unit, wherein the at least one on-board communication unit is adapted to wirelessly receive commands for the passive microwave radiometer from a ground based communication unit. The on-board communication unit and the ground based communication unit are preferably connected by a wireless network. The ground based communication unit is preferably a personal computer, laptop, or smart phone.

This use of an aerial drone for detecting moisture in a roof structure reduces the need for workers to be on roofs. Furthermore, the process for detecting moisture can be fully or at least partially automated. This reduces the risk for the worker, who can work from the ground using the ground-based communication unit.

In one exemplary embodiment, the aerial drone comprises at least one on-board communication unit, wherein the at least one on-board communication unit is adapted to receive recordings from the passive microwave radiometer, for example, via a computer unit of the radiometer electronics, and to wirelessly send the recordings to a ground based communication unit. The at least one on-board communication unit allows that recordings from the passive microwave radiometer can be sent to a ground based communication unit.

In a further exemplary embodiment, the aerial drone comprises at least one on-board communication unit, wherein the at least one on-board communication unit is adapted to wirelessly receive commands for the passive microwave radiometer from a ground based communication unit and to transfer the commands to the passive microwave radiometer and wherein the at least one on-board communication unit is adapted to receive recordings from passive microwave radiometer and to wirelessly send the recordings to a ground based communication unit. The combination of these features allows a good observation and control of the moisture detection process from a ground based communication unit.

In one exemplary embodiment, aerial drone comprises one camera and one on-board communication unit. These embodiments save weight, which allows a longer flight time for the aerial drone and/or saves weight for other features.

The aerial drone including the passive microwave radiometer, at least one camera, and at least one on-board communication unit may have a weight of 1 kg to 25 kg, preferably 3 kg to 15 kg, more preferably 5 kg to 10 kg. Such aerial drones have been found out have a sufficient operation/flight time when equipped with a standard portable power supply, particularly rechargeable or replaceable batteries.

In an exemplary embodiment, the aerial drone comprises at least one battery unit with a sum of electric charge of 14000 mAh to 30000 mAh, preferably 19000 mAh to 27000 mAh, for providing power to at least one motor of the drone.

The at least one on-board communication unit may be a microcomputer, preferably a single-board computer or a Raspberry Pi, with an operating system. A microcomputer, such as a single-board computer or a Raspberry Pi, is very light, requires little space and can be easily attached to the aerial drone. Further, these require only a small and light battery, which further reduces the weight of the aerial drone.

In one embodiment, moisture is detected in a roof structure comprising a roof substrate and a roofing membrane covering the roof substrate.

Generally, any type of the roofing membrane can be used in the roof structure. Typical roofing membranes used in flat roof systems include single- and multi-ply polymeric membranes based on thermoplastic materials such as TPOs (thermoplastic olefins) and plasticized polyvinylchloride (p-PVC) or chemically crosslinked elastomers such as ethylene propylene diene monomer (EPDM). However, liquid applied membranes (LAMs) can also be used as the roofing membrane.

The roof substrate may comprise at least one of a roof deck, a vapor control membrane, an insulation board, and a cover board.

Suitable insulation boards include foamed boards that are typically composed of expanded polystyrene (EPS), extruded expanded polystyrene (XPS), or polyisocyanurate (PIR). The roofing membrane can be installed to cover the upper surface of the insulation board. Insulation boards based on foamed synthetic organic polymers have relatively low compression strength and, therefore, cover boards (also known as hardboards) are commonly used, especially in areas where the roof structure is exposed to harsh environmental conditions, to improve the resistance of the insulation board against mechanical impacts, especially hail storms. In these roof structures, the roofing membrane is installed over the cover board. A vapor control membrane is typically installed below the insulation board to control the movement of water through the roof structure by vapor diffusion.

Another aspect of the present invention is use of a passive microwave radiometer for detecting of leakages in a roof structure.

In one preferred embodiment, the passive microwave radiometer is a dual-polarization L-band radiometer, preferably a portable dual-polarization L-band radiometer.

In one exemplary embodiment, the passive microwave radiometer used for detecting of leakages in a roof structure is the passive microwave radiometer used in the method for detecting moisture in a roof structure as discussed above.

Furthermore, the passive microwave radiometer may be integrated in an unmanned aerial vehicle (UAV), movable cart, or a hand-held device.

In one exemplary embodiment, the passive microwave radiometer is integrated into an unmanned aerial vehicle (UAV), preferably an aerial drone.

Preferred embodiments for the aerial drone with an integrated passive microwave radiometer have already been discussed above.

### Examples

### Detecting moisture under roofing membranes

A portable L-band microwave radiometer (PoLRa) comprising a patch array antenna provided by TerraRad Tech AG was used to measured microwave emissions of a roof structure. The portable radiometer was installed to a front side of movable cart with wheels such that the patch array formed an angle of ca. 45° with the horizontal plane of the roof structure. The movable cart was equipped with a GPS module having a measurement accuracy of ca. 1.5 m.

The tested roof structures included a mineral wool or expanded polystyrene (EPS) insulation board and a roofing membrane covering the upper surface of the insulation board.

In the measurements, each longitudinal portion (lanes 1 to 3) of the roofing membrane was passed one or more times to measure the brightness temperatures at horizontal (TBH) and vertical polarizations (THV).

Four types of setups were used to test the ability of the microwave radiometer to detect moisture in a roof structure:
1) PVC-based roofing membrane with one size of wet spot under the membrane
2) TPO-based roofing membrane with one size of wet spot under the membrane
3) TPO-based roofing membrane with three different sizes of wet spots (small, medium, large) under the membrane
4) Folded TPO-based roofing membrane with a one size wet spot under the membrane.

In the fourth set up, the underside of the roofing membrane was wetted on one side near the left longitudinal edge of the membrane while the side near the right longitudinal edge was left dry.

In the first set-up, the same lane of the roofing membrane was passed twice with the movable cart to measure the brightness temperatures whereas in the second, third, and fourth set-up, the same lane was passed only once.

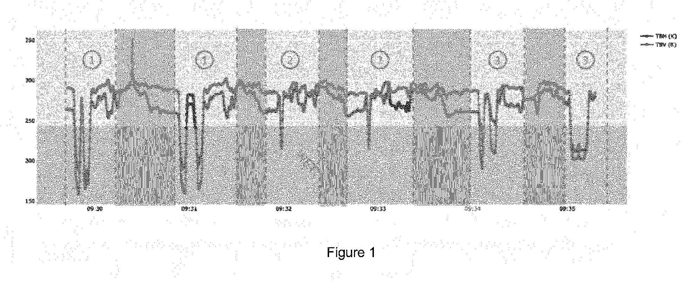

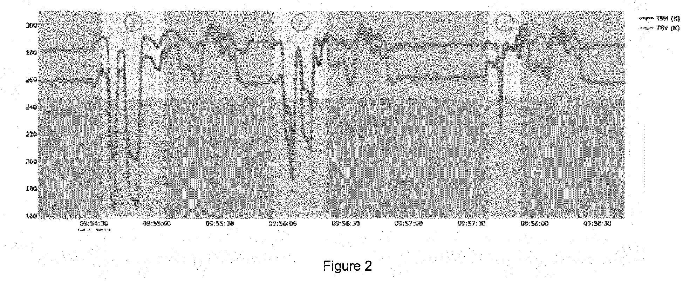

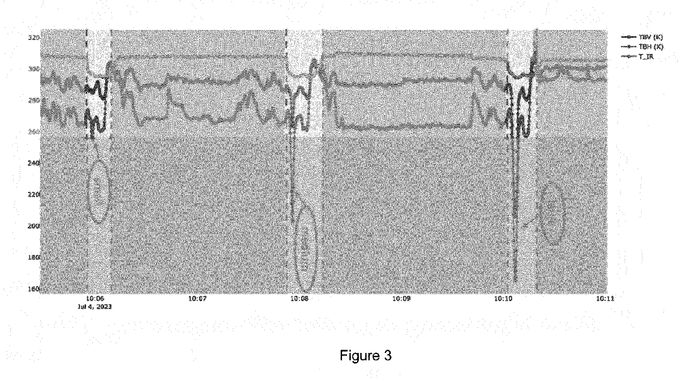

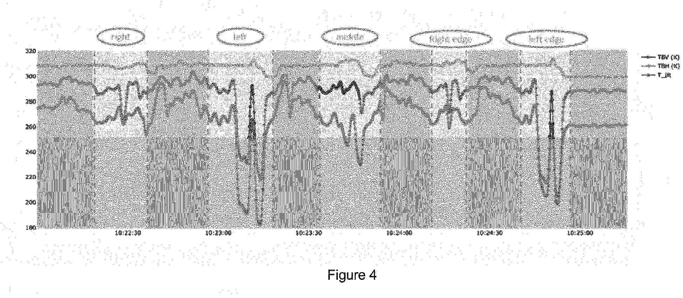

The measured brightness temperatures (TBH, TBV) are shown in Figures 1-4. In of the tested each set-ups, the location of the moisture (wet spot) under the roofing membrane could be detected with the accuracy of the GPS location method based on a threshold value or peaks (valleys) of the measured brightness temperatures. The threshold brightness temperature for the wet spot was 245 K for the 1^{st} and 2^{nd} set-up, 255 K for the 3^{rd} set-up, and 250 K for the 4^{th} set-up. Measured brightness temperatures below the threshold values indicated a presence of moisture under the roofing membrane.

## Claims

1. A method for detecting moisture in a roof structure comprising measuring microwave emission of the roof structure.

2. The method according to claim 1, wherein the measurement of microwave emission of the roof structure is conducted using a passive microwave radiometer.

3. The method according to claim 2, wherein the passive microwave radiometer is a dual-polarization L-band radiometer, preferably a portable dual-polarization L-band radiometer.

4. The method according to any one of previous claims comprising measuring dual polarization microwave brightness temperatures and using retrieval algorithms based on microwave emission models to determine the moisture content in the roof structure.

5. The method according to claim 4, wherein the brightness temperatures are measured at nadir angles at horizontal and vertical polarizations (H, V).

6. The method according to claim 4 or 5, wherein an emission model is used in the retrieval algorithm, wherein the emission model is preferably selected from tau-omega (TO) and multiple-scattering two-stream (2S) models.

7. The method according to any one of claims 2-6, wherein the passive microwave radiometer comprises a patch array antenna, preferably comprising at least one patch array with a patch substrate layer as printed circuit board of a dielectric material and a pattern of printed patches on a front side of the patch substrate layer.

8. The method according to any one of claims 2-7, wherein the passive microwave radiometer is integrated in an unmanned aerial vehicle (UAV), movable cart, or a hand-held device.

9. The method according to any one of claims 2-8, wherein the passive microwave radiometer is integrated in an unmanned aerial vehicle (UAV), preferably an aerial drone, preferably comprising at least one camera and at least one on-board communication unit.

10. The method according to any one of previous claims, wherein the roof structure comprises a roof substrate and a roofing membrane covering the roof substrate.

11. The method according to claim 11, wherein the roof substrate comprises at least one of a roof deck, an insulation board, and a cover board.

12. Use of a passive microwave radiometer for detecting of leakages in a roof structure.

13. The use according to claim 12, wherein the passive microwave radiometer is a dual-polarization L-band radiometer, preferably a portable dual-polarization L-band radiometer.

14. The use according to claim 12 or 13, wherein the passive microwave radiometer comprises a patch array antenna, preferably comprising at least one patch array with a patch substrate layer as printed circuit board of a dielectric material and a pattern of printed patches on a front side of the patch substrate layer.

15. The use according to any one of claims 12-14, wherein the passive microwave radiometer is integrated in an unmanned aerial vehicle (UAV), a movable cart, or a hand-held device.
